(19) Europäisches Patentamt
European Patent Office
Office européen des brevets

(11) Veröffentlichungsnummer: **0 003 144**
**B1**

(12) # EUROPÄISCHE PATENTSCHRIFT

(45) Veröffentlichungstag der Patentschrift: **05.08.81**

(21) Anmeldenummer: **79100149.8**

(22) Anmeldetag: **18.01.79**

(51) Int. Cl.³: **C 07 D 213/80,** C 07 D 401/04, C 07 D 211/90, A 61 K 31/455 // C07C69/76, C07C69/95, C07C101/34

(54) **4-Pyridon-3-carbonsäurederivate, diese enthaltende pharmazeutische Präparate und deren Herstellung sowie deren Verwendung.**

(30) Priorität: **18.01.78 CH 512/78**
**15.11.78 CH 11734/78**

(43) Veröffentlichungstag der Anmeldung: **25.07.79 Patentblatt 79/15**

(45) Bekanntmachung des Hinweises auf die Patenterteilung: **05.08.81 Patentblatt 81/31**

(84) Benannte Vertragsstaaten: **IT**

(56) Entgegenhaltungen:
**JOURNAL OF HETEROCYCLIC CHEMISTRY 14 (1) Februar 1977, Utah, TETSUJI KAMETANI et al.: "Synthetic studies on chemotherapeutics II (1) Synthesis of phenylsubstituted 1,4 dihydro-4-oxonicotinic acid derivatives", Seiten 477-482**

(73) Patentinhaber: **F. HOFFMANN-LA ROCHE & CO. Aktiengesellschaft, CH-4002 Basel (CH)**

(72) Erfinder: **Wick, Alexander Eduard, Dr., Mühlestiegstrasse 34, CH-4125 Riehen (CH)**

(74) Vertreter: **Lederer, Franz, Dr. et al, Patentanwälte Lederer, Meyer Lucile-Grahn-Strasse 22, D-8000 München 80 (DE)**

**EP 0 003 144 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 4-Pyridon-3-carbonsäurederivate, diese enthaltende pharmazeutische Präparate und deren Herstellung sowie deren Verwendung

Die vorliegende Erfindung betrifft neue 4-Pyridon-3-carbonsäurederivate und pharmazeutische Präparate mit einem Gehalt an diesen Verbindungen sowie deren Herstellung.

Die neuen Verbindungen sind antibakteriell aktiv und/oder zeigen eine Wirkung auf das Zentralnervensystem, wobei sie gegenüber bekannten Verbindungen gleicher Wirkungsrichtung, z.B. gegenüber Nitrofurantoin oder Nomifensin, Vorteile aufweisen.

Die erfindungsgemässen 4-Pyridon-3-carbonsäurederivate sind solche der allgemeinen Formel

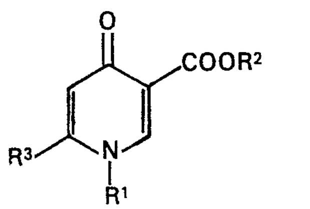

I

worin

$R^1$ $C_{1-8}$-Alkyl, $C_{3-10}$-Cycloalkyl, $C_{3-10}$-Cycloalkyl-$C_{1-6}$-alkyl, $C_{1-6}$-Alkoxy oder $C_{1-6}$-Alkoxy-$C_{1-6}$-alkyl;

$R^2$ Wasserstoff

$R^3$ einen ggf. durch $R^4$, $R^5$ und/oder $R^6$ substituierten Phenyl-, Phenäthyl- oder Styrylrest oder einen ggf. durch $R^7$ substituierten, ein oder mehrere N-Atome enthaltenden aromatisch heterocyclischen 6-Ring, der über ein Ring-C-atom gebunden ist;

$R^4$ und $R^5$ Halogen, Trifluormethyl, Hydroxy, $C_{1-6}$-Alkoxy, $C_{1-6}$-Alkylthio, $C_{1-6}$-Alkyl, Amino, Acylamino, $C_{1-6}$-Alkylamino, Di-$C_{1-6}$-alkylamino, Nitro, einen über ein Ring-N- oder -C-atom gebundenen 5- oder 6-gliedrigen Heterocyclus oder beide Reste $R^4$ und $R^5$ gemeinsam $C_{1-6}$-Alkylendioxy;

$R^6$ $C_{1-6}$-Alkoxy und

$R^7$ Halogen, Hydroxy, $C_{1-6}$-Alkoxy, $C_{1-6}$-Alkylthio, $C_{1-6}$-Alkyl, Amino, $C_{1-6}$-Alkylamino oder Di-$C_{1-6}$-alkylamino bedeuten,

- mit Ausnahme von 1-Methyl-4-oxo-6-phenyl-1,4-dihydro-nicotinsäure und deren Salze.

Aus J. Heterocyclic Chemistry 14, 477–482 [1977] sind den erfindungsgemässen Verbindungen der Formel I strukturell nahestehende Verbindungen und deren Herstellung bekannt, nämlich 1-Ethyl-4-oxo-5-(methyl oder phenyl)-6-(phenyl oder methyl)-1,4-dihydro-nicotinsäure und deren Ethylester, ohne dass Angaben über deren pharmakologische Wirkung gemacht wurden. Vielmehr wurde nur darauf hingewiesen, dass Untersuchungen über deren antibakterielle Aktivitäten im Gange seien. Andererseits sind aus der U.S. Patentschrift 4.152.136 in 5-Stellung substituierte 4-Oxo-1,4-dihydronicotinsäureester bekannt, die Stellungsisomere der entsprechenden Ester der erfindungsgemässen Verbindungen der Formel I sind und die keine antibakterielle Aktivität aufweisen. Ebenso weisen die entsprechenden, in 5-Stellung substituierten 4-Oxo-1,4-dihydronicotinsäuren keine antibakterielle Aktivität auf. Angesichts dieses Standes der Technik musste es überraschen, dass die in 6-Stellung substituierten 1,4-Dihydronicotinsäurederivate der vorliegenden Erfindung pharmakologisch aktiv sind.

Die folgende Tabelle mit einer Gegenüberstellung der minimalen Hemmkonzentrationen (MIC) gegen E. coli zweier repräsentativer Verbindungen (A und B) und ihrer Stellungsisomeren (C und D) verdeutlicht diesen Sachverhalt:

| Verbindung | MIC [µg/ml] E. coli 1346 |
|---|---|
| 1-Äthyl-6-phenyl-4-oxo-1,4-dihydronicotinsäure (A) | 10 |
| 1-Äthyl-6-(3,4-dimethoxyphenyl)-4-oxo-1,4-dihydronicotinsäure (B) | 78 |
| 1-Äthyl-5-phenyl-4-oxo-1,4-dihydronicotinsäure (C) | 312 |
| 1-Äthyl-5-(3,4-dimethoxyphenyl)-4-oxo-1,4-dihydronicotinsäure (D) | 312 |

Das erfindungsgemässe Verfahren ist dadurch gekennzeichnet, dass man

(a) ein 1,4,5,6-Tetrahydronicotinsäurederivat der Formel

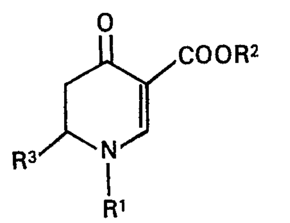

II

worin $R^1$, $R^2$ und $R^3$ die oben angegebenen Bedeutungen besitzen,

in 5,6-Stellung dehydriert oder

(b) einen entsprechenden 1,6-disubstituierten 4-Oxo-1,4-dihydronicotinsäureester verseift oder

(c) einen Substituenten $R^3$ innerhalb der oben angegebenen Definitionen in an sich bekannter Weise modifiziert

und gewünschtenfalls die erhaltene Verbindung der Formel I in ein Salz überführt.

Beispiele von $C_{1-8}$-Alkylgruppen, die gerad- oder verzweigtkettig sein können, sind Methyl, Äthyl, Propyl, Isopropyl, Butyl, sek.- oder tert.-Butyl und Octyl, wobei Methyl und Äthyl bevorzugt sind; Beispiele von $C_{1-6}$-Alkylendioxy sind Methylen- und Äthylendioxy. Als $C_{3-10}$-Cycloalkylrest ist Cyclopropyl bevorzugt. Der Ausdruck Halogen umfasst Fluor, Chlor, Brom und Jod. Beispiele für den Substituenten $R^3$ in der Bedeutung eines ein oder mehrere N-Atome enthaltenden aromatischen heterocyclischen 6-Ringes sind 2-, 3- oder 4-Pyridyl, 3- oder 4-Pyridazyl, 2-, 4- oder

5-Pyrimidyl und 2- oder 3-Pyrazyl, während als Substituenten $R^4$ und/oder $R^5$ in der Bedeutung eines über ein Ring-N- oder -C-Atom gebundenen 5- oder 6-gliedrigen Heterocyclus' beispielsweise Pyrrol, ein Pyrrolin, Pyrrolidin, Isoxazol, Oxazol, Thiophen, Thiazol, Pyrazol, Imidazol, ein Imidazolin, Imidazolidin, Triazol, Oxadiazol, Pyridin, Piperidin, Morpholin, Piperazin, Thiazin, Pyridazin, Pyrimidin oder ein Triazin genannt werden können. Die Acylgruppe in einem Acylaminorest leitet sich vorzugsweise von einer aliphatischen Carbonsäure, insbesondere einer solchen mit 1–6 C-Atomen, z.B. der Ameisen-, Essig- oder Propionsäure, ab.

Als Salze von Verbindungen der Formel I kommen Alkalimetall-, Erdalkalimetall- und (ggf. substituierte) Ammoniumsalze in Frage, wenn $R^2$ Wasserstoff bedeutet und, sofern basische Substituenten im Molekül vorliegen, Additionssalze mit physiologisch verträglichen, starken anorganischen und organischen Säuren, wie z.B. Salzsäure, Schwefelsäure, Phosphorsäure, Methansulfonsäure oder p-Toluolsulfonsäure.

Bevorzugte Verbindungen der Formel I sind einerseits solche, in denen $R^1$ Methyl oder Äthyl ist, andererseits solche, in denen $R^3$ einen substituierten Phenylrest darstellt, wobei ein Substituent, insbesondere eine substituierte Aminogruppe oder ein stickstoffhaltiger Heterocyclus, in 4-Stellung besonders bevorzugt ist.

Die erfindungsgemässe Dehydrierung einer Verbindung II gemäss Verfahrensvariante (a) kann nach an sich bekannten Methoden, zweckmässigerweise mit einem substituierten Benzochinon, wie 2,3-Dichlor-5,6-dicyan-1,4-benzochinon (DDQ) oder Tetrachlor-1,4-benzochinon (Chloranil), in einem inerten organischen Lösungsmittel, wie Methylenchlorid, Benzol oder Dioxan, und innerhalb eines Temperaturbereichs von Raumtemperatur bis zur Rückflusstemperatur des Reaktionsgemisches durchgeführt werden. Im allgemeinen geht man so vor, dass man zu der Lösung des Tetrahydronicotinsäurederivats eine Lösung des substituierten Benzochinons zutropft, wobei man darauf achtet, dass äquimolekulare Mengen umgesetzt werden. Bei ungefärbtem Ausgangsmaterial ist das Ende der Reaktion leicht durch die auftretende Färbung des Reaktionsgemisches zu erkennen. Das Reaktionsprodukt kann in üblicher Weise aus dem Gemisch isoliert und z.B. durch Umkristallisation oder chromatographische Verfahren gereinigt werden.

Die Verseifung eines 4-Oxo-1,4-dihydronicotinsäureesters gemäss Verfahrensvariante (b) kann in an sich bekannter Weise erfolgen.

Gemäss Verfahrensvariante (c) können die Substituenten $R^2$ und $R^3$ in an sich bekannter Weise innerhalb der angegebenen Definitionen modifiziert werden.

So kann beispielsweise eine im Substituenten $R^3$ vorkommende Aminogruppe alkyliert, eine Nitro- zu einer Aminogruppe reduziert, eine Hydroxygruppe veräthert, eine Alkoxygruppe gespalten, ein Halogenatom gegen ein anderes Halogenatom oder eine andere funktionelle Gruppe ausgetauscht werden. Ferner kann ein gesättigter heterocyclischer Substituent gewünschtenfalls dehydriert, ein teilweise oder völlig ungesättigter Heterocyclus oder der Styrylrest zum Phenäthylrest hydriert werden, ohne dass dadurch die allen erfindungsgemässen Verbindungen gemeinsame Grundstruktur verändert wird.

Die Ausgangsverbindungen der Formel II können erfindungsgemäss durch Erwärmen eines 2-($R^1$-aminomethylen)-3-oxo-4-pentensäurederivates der Formel

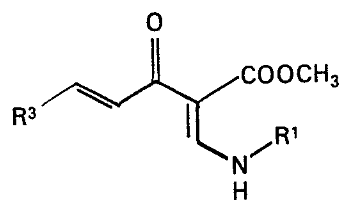

III

worin $R^1$ und $R^3$ die oben angegebenen Bedeutungen haben,

zweckmässigerweise in einem inerten polaren aprotischen organischen Lösungsmittel, wie z.B. Dimethylformamid (DMF), Dimethylsulfoxid (DMSO) oder Hexamethylphosphorsäuretriamid, hergestellt werden. Die Lösung wird eine bis mehrere Stunden auf eine Temperatur von etwa 100 °C bis zur Rückflusstemperatur erhitzt, wobei Ringschluss zum 1,4,5,6-Tetrahydropyridon erfolgt.

Die Herstellung der Verbindungen III wiederum kann in an sich bekannter Weise in einer mehrstufigen Synthese aus Aldehyden der Formel $R^3$-CHO erfolgen.

Im folgenden Reaktionsschema (Seite 4) ist die Herstellung von 1-Äthyl-6-phenyl-4-oxo-1,4-dihydronicotinsäure aus Benzaldehyd generisch und in Beispiel 1 die Herstellung von 1-Äthyl-6-(4-chlorphenyl)-4-oxo-1,4-dihydronicotinsäure bzw. deren Methylester im Detail beschrieben.

Durch Variation des Ausgangsaldehyds VII-1, der Estergruppe der Triphenylphosphoniumverbindung oder des Amins bei der Umsetzung einer Verbindung IV-1 zu einer Verbindung III-1 können in analoger Weise prinzipiell alle erfindungsgemässen Verbindungen hergestellt werden.

Die Verbindungen der Formel I sind pharmakologisch aktiv. Sie weisen antibakterielle Aktivität und/oder eine stimulierende Wirkung auf das Zentralnervensystem auf, wobei sie durch geringe akute Toxizitäten gekennzeichnet sind. So ist beispielsweise 1-Äthyl-4-oxo-6-phenyl-1,4-dihydronicotinsäure dem Nomifensin, bei etwa gleicher $LD_{50}$, wirkungsmässig wesentlich überlegen, während es sich vom d-Amphetamin und d-Methamphetamin bei etwa gleich starker Wirkung durch die wesentlich niedrigere $LD_{50}$ vorteilhaft abhebt (s. Tabelle I).

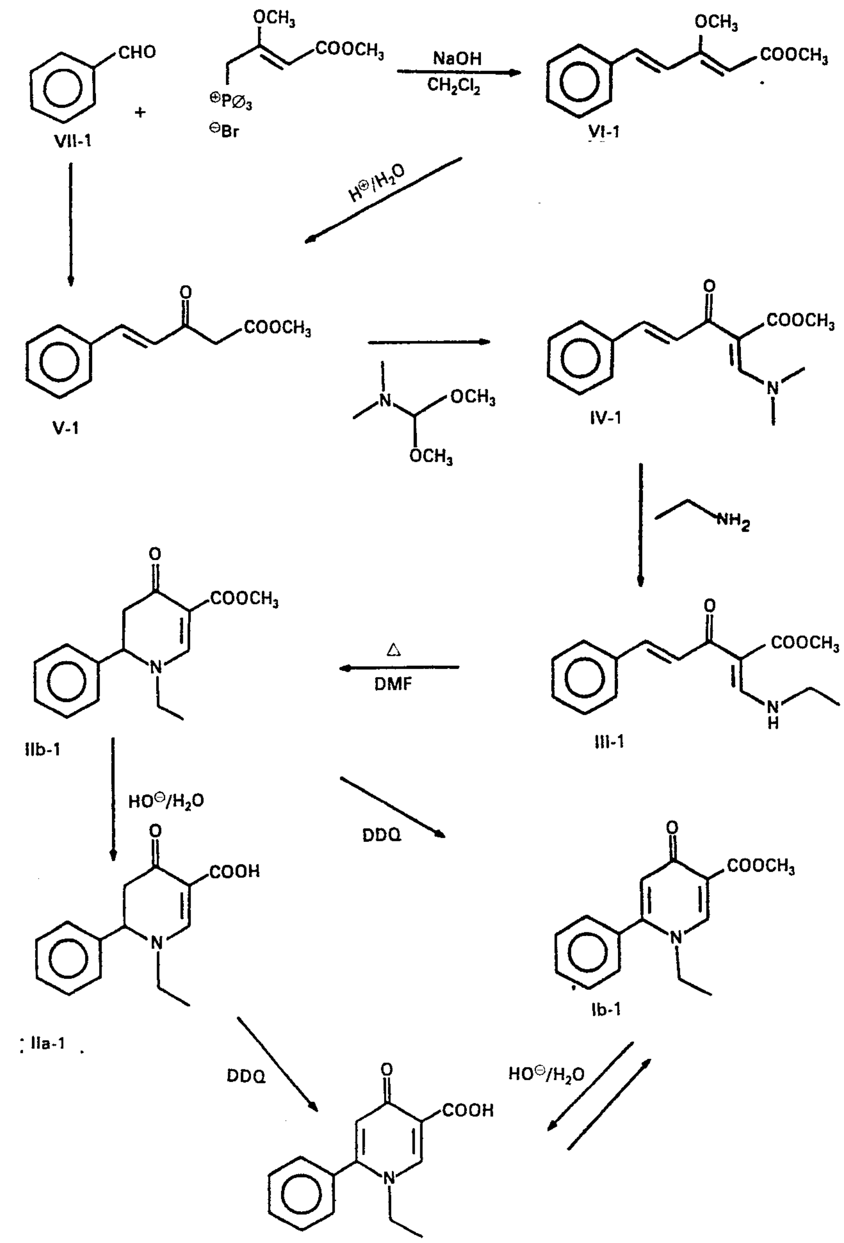


Tabelle I

| Verbindung | $LD_{50}$ | Turning rat-Test[1] min. aktive Dosis [mg/kg] i.p. |
|---|---|---|
| 1-Äthyl-4-oxo-6-phenyl-1,4-dihydronicotinsäure | 300–600 p.o. | 1 |
| Nomifensin | 300–600 p.o. | 3 |
| d-Amphetamin. 1/2 $H_2SO_4$ | 35 p.o., 14 i.v., 22 s.c. | 1 |
| d-Methamphetamin. HCl | 9.5 i.v., 14 s.c. | 1 |

[1] Arch. int. Pharmacodyn. Ther. 217, 118–130 (1975)

Was die antibakterielle Aktivität der neuen Verbindungen angeht, so eignen sie sich besonders als Therapeutika bei Harnweginfektionen. Gegenüber bekannten Verbindungen mit dieser Indikation, z.B. gegenüber Nitrofurantoin, zeichnen sie sich durch erhöhte Aktivität gegenüber speziellen Erregern, beispielsweise Escherichia coli, aus (s. Tabelle 2).

Tabelle 2

| Verbindung | Escherichia coli in vitro MIC µg/ml | Escherichia coli in vivo (Maus) $ED_{50}$ mg/kg p.o. |
|---|---|---|
| Nitrofurantoin | 5 | >100 |
| 1-Äthyl-6-(4-methyloxyphenyl)-4-oxo-1,4-dihydronicotinsäure | 2,5 | >100 |
| 1-Äthyl-6-(3,4-methylendioxyphenyl)-4-oxo-1,4-dihydronicotinsäure | 2,5 | >100 |
| 1-Äthyl-6-(4-methylthiophenyl)-4-oxo-1,4-dihydronicotinsäure | 2,5 | 82 |
| 1-Äthyl-6-[4-(1-pyrrolyl)phenyl]-4-oxo-1,4-dihydronicotinsäure | 1,2 | 34 |
| 1-Äthyl-6-[4-(1-pyrrolidinyl)phenyl]-4-oxo-1,4-dihydronicotinsäure | 1,2 | 19 |
| 1-Äthyl-6-(4-dimethylaminophenyl)-4-oxo-1,4-dihydronicotinsäure | 0,6 | 8,8 |
| 1-Äthyl-6-(3-methyl-4-methylamino phenyl)-4-oxo-1,4-dihydronicotinsäure | 0,6 | 3,5 |

Die erfindungsgemässen 4-Pyridon-3-carbonsäurederivate der Formel I können daher bei der Therapie und Prophylaxe von Krankheiten, insbesondere von bakteriellen Infektionen, sowie zur Stimulierung des Zentralnervensystems in Form pharmazeutischer Präparate mit direkter oder verzögerter Freigabe des Wirkstoffes in Mischung mit einem für die orale, rektale oder parenterale Applikation geeigneten organischen oder anorganischen inerten Trägermaterial, z.B. Wasser, Gelatine, Gummi arabicum, Milchzukker, Stärke, Magnesiumstearat, Talk, pflanzlichen Ölen, Polyalkylenglykolen, Vaseline®, usw. verwendet werden. Die pharmazeutischen Präparate können in fester Form, z.B. als Tabletten, Dragées, Suppositorien, Kapseln; in halbfester Form, z.B. als Salben; oder in flüssiger Form, z.B. als Lösungen, Suspensionen oder Emulsionen, vorliegen. Gegebenenfalls sind sie sterilisiert und bzw. oder enthalten weitere Hilfsstoffe, wie Konservierungs-, Stabilisierungs-, Netz- oder Emulgiermittel, Mittel zur geschmacklichen Verbesserung, Salze zur Veränderung des osmotischen Druckes oder Puffersubstanzen.

Die Herstellung der pharmazeutischen Präparate kann in der jedem Fachmann geläufigen Weise, nämlich dadurch erfolgen, dass man die Wirksubstanz mit dem zur therapeutischen Verabreichung geeigneten, nicht-toxischen, inerten Trägermaterial vermischt und das erhaltene Gemisch in die geeignete galenische Form bringt.

Als Dosierungsrichtlinie kann für die Verbindungen der Formel I als antibakterielle Mittel eine Menge von 10–100 mg/kg, vorzugsweise etwa 50 mg/kg Körpergewicht pro Tag gelten, als Mittel mit Wirkung auf das Zentralnervensystem eine Menge von 100 µg–10 mg/kg.

Beispiel 1

3 g 1-Äthyl-6-(4-chlorphenyl)-4-oxo-1,4,5,6-tetrahydronicotinsäure-methylester wurden in 50 ml Benzol auf 80° erwärmt und tropfenweise mit einer benzolischen Lösung von 4 g Dichlordicyanbenzochinon (DDQ) versetzt, bis keine Entfärbung mehr sichtbar war. Es wurde gekühlt und das dabei ausfallende kristalline Material abgenutscht.

Das erhaltene Material, ein Gemisch von 1-Äthyl-6-(4-chlorphenyl)-4-oxo-1,4-dihydronicotinsäuremethylester mit DDQ, wurde mit 20 ml 1 N Natronlauge bei Raumtemperatur während 30 Minuten gerührt, die klare Lösung mit 50 ml Eiswasser verdünnt und mit 0,5 N Salzsäure vorsichtig auf pH 6,5 angesäuert. Dabei fiel das Produkt in fast reiner Form an. Es wurde aus Essigester umkristallisiert. Ausbeute: 2,1 g 1-Äthyl-6-(4-chlorphenyl)-4-oxo-1,4-dihydronicotinsäure, Smp. 215–217°C.

Das Ausgangsmaterial wurde folgendermassen erhalten:

14 g 4-Chlorbenzaldehyd und 47 g ‹2-Methoxy-3-(methoxy-carbonyl)-allyl›-triphenylphosphoniumbromid wurden in 130 ml Methylenchlorid gelöst. Dazu liess man bei Raumtemperatur und unter gutem Rühren 100 ml 50% wässrige Natronlauge zufliessen, wobei ein Temperaturanstieg beobachtet wurde. Es wurde 20 Minuten weitergerührt, auf Eis gegossen und mit Methylenchlorid extrahiert. Der nach Trocknen mit Natriumsulfat und Absaugen des Lösungsmittels verbleibende Rückstand wurde aus Methanol kristallisiert, die Mutterlauge in Hexan/Äther (4:1 v/v), durch eine kurze Silicagelsäule filtriert und das aus dem Eluat auskristallisierte Produkt mit dem Hauptprodukt vereinigt. Ausbeute insgesamt:

23,5 g (93,3%) reiner 5-(4-Chlorphenyl)-3-methoxy-2,4-pentadiencarbonsäuremethylester, Smp. 69–72 °C.

23,5 g 5-(p-Chlorphenyl)-3-methoxy-2,4-pentadiencarbonsäuremethylester, gelöst in 250 ml Dioxan, wurden mit 150 ml 0,1 N Schwefelsäure versetzt und während 3 Stunden auf 100 °C gehalten. Man kühlte ab, extrahierte mit Essigester und kristallisierte nach Trocknen der Lösung durch Zugabe von Hexan. Man erhielt 14,8 g (66,7%) 5-(4-Chlorphenyl)-3-oxo-4-pentensäuremethylester, Smp. 78–80 °C.

14,8 g 5-(4-Chlorphenyl)-3-oxo-4-pentensäuremethylester wurden in 100 ml Benzol gelöst und mit N,N-Dimethylformamid-dimethylacetal versetzt. Man rührte die rotbraun gefärbte Lösung während 30 Minuten bei 60 °C, entfernte Lösungsmittel und überschüssiges Reagens und erhielt 5-(4-Chlorphenyl)-2-(dimethylaminomethylen)-3-oxo-4-pentensäuremethylester in Form eines rotbraunen Öles, das in dieser Form weiterverarbeitet wurde.

Das erhaltene Öl wurde in 50 ml Benzol gelöst und mit 100 ml einer gesättigten benzolischen Lösung von Äthylamin bei Raumtemperatur 30 Minuten gerührt. Danach wurde das Lösungsmittel entfernt und der Rückstand aus Äther/Hexan kristallisiert. Man erhielt 12,4 g (68% über 2 Stufen) 2-(Äthylaminomethylen)-5-(4-chlorphenyl)-3-oxo-4-pentensäuremethylester, Smp. 78–80 °C.

5,2 g 2-(Äthylaminomethylen)-5-(4-chlorphenyl)-3-oxo-4-pentensäuremethylester in 50 ml Dimethylformamid wurden während 3 Stunden bei 140–150 °C gehalten. Nach Entfernung des Lösungsmittels wurde 1-Äthyl-6-(4-chlorphenyl)-4-oxo-1,4,5,6-tetrahydronicotinsäuremethylester als einheitlicher, öliger Rückstand erhalten.

In analoger Weise wurden erhalten

- aus Benzaldehyd und Propylamin 4-Oxo-6-phenyl-1-propyl-1,4-dihydronicotinsäure, Smp. 153–154 °C;
- aus Benzaldehyd und Isopropylamin 1-Isopropyl-4-oxo-6-phenyl-1,4-dihydronicotinsäure, Smp. 215–216 °C;
- aus Benzaldehyd und Butylamin 1-Butyl-4-oxo-6-phenyl-1,4-dihydronicotinsäure, Smp. 119–120 °C;
- aus Benzaldehyd und tert.-Butylamin 1-tert.-Butyl-4-oxo-6-phenyl-1,4-dihydronicotinsäure, Smp. 295 °C (Zers.);
- aus Benzaldehyd und Octylamin 1-Octyl-4-oxo-6-phenyl-1,4-dihydronicotinsäure, Smp. 62–63 °C;
- aus Benzaldehyd und Cyclopropylmethylamin 1-(Cyclopropylmethyl)-4-oxo-6-phenyl-1,4-dihydronicotinsäure, Smp. 177–179 °C;
- aus Benzaldehyd und Cyclopropylamin 1-Cyclopropyl-4-oxo-6-phenyl-1,4-dihydronicotinsäure, Smp. 180–181 °C;
- aus Benzaldehyd und Cyclopentylamin 1-Cyclopentyl-4-oxo-6-phenyl-1,4-dihydronicotinsäure, Smp. 188–190 °C;
- aus Benzaldehyd und Cyclohexylamin 1-Cyclohexyl-4-oxo-6-phenyl-1,4-dihydronicotinsäure, Smp. 257–259 °C;
- aus Benzaldehyd und 1-Adamantylamin 1-(1-Adamantyl)-4-oxo-6-phenyl-1,4-dihydronicotinsäure, Smp. 248 °C (Zers.);
- aus Benzaldehyd und 2-Methoxyäthylamin 1-(2-Methoxyäthyl)-4-oxo-6-phenyl-1,4-dihydronicotinsäure, Smp. 144–146 °C;
- aus Benzaldehyd und Methoxylamin 1-Methoxy-4-oxo-6-phenyl-1,4-dihydronicotinsäure, Smp. 193–194 °C;
- aus Benzaldehyd und O-Butyl-hydroxylamin 1-Butyloxy-4-oxo-6-phenyl-1,4-dihydronicotinsäure, Smp. 130–132 °C;
- aus 3-Nitrobenzaldehyd und Äthylamin 1-Äthyl-6-(3-nitrophenyl)-4-oxo-1,4-dihydronicotinsäure, Smp. 242–243 °C;
- aus 3-Aminobenzaldehyd und Äthylamin 1-Äthyl-6-(3-aminophenyl)-4-oxo-1,4-dihydronicotinsäure, Smp. 145–146 °C;
- aus 4-(Dimthylamino)-benzaldehyd und Methoxylamin 6-[4-(dimethylamino)-phenyl]-1-methoxy-4-oxo-1,4-dihydronicotinsäure, Smp. 224 °C (Zers.);
- aus 4-(Dimethylamino)-benzaldehyd und O-Butylhydroxylamin 1-Butoxy-6-[4-(dimethylamino)-phenyl]-4-oxo-1,4-dihydronicotinsäure, Smp. 155–157 °C;
- aus 4-Acetamidobenzaldehyd und Äthylamin 6-(4-Acetamidophenyl)-1-äthyl-4-oxo-1,4-dihydronicotinsäure, Smp. 237,5–239 °C;
- aus 3-(Trifluormethyl)-benzaldehyd und Äthylamin 1-Äthyl-4-oxo-6-[3-(trifluormethyl)-phenyl]-1,4-dihydronicotinsäure, Smp. 186–188 °C;
- aus 4-(4-Methylpiperazino)-benzaldehyd und Äthylamin 1-Äthyl-6-[4-(4-methyl-1-piperazinyl)-phenyl]-4-oxo-1,4-dihydronicotinsäure, Smp. 246–248 °C;
- aus p-Methoxyzimtsäurealdehyd und Äthylamin 1-Äthyl-6-(p-methoxystyryl)-4-oxo-1,4-dihydronicotinsäure, Smp. 237–238 °C.

Beispiel 2

800 mg 1-Äthyl-6-(4-chlorphenyl)-4-oxo-1,4,5,6-tetrahydronicotinsäure in 20 ml Benzol wurden bei 80 °C tropfenweise mit einer Lösung von 1,5 g DDQ in 30 ml Benzol versetzt, bis sich die Lösung nicht mehr entfärbte. Man kühlte ab, nutschte den Niederschlag ab, löste diesen in 20 ml 1N Natronlauge auf und säuerte die Lösung vorsichtig mit kalter 0,5 N Salzsäure auf pH 6,5 an. Das ausfallende, farblose Produkt wurde mit Wasser und dann mit Äther gewaschen. Ausbeute 0,7 g (88%) 1-Äthyl-6-(4-chlorphenyl)-4-oxo-1,4-dihydronicotinsäure, Smp. 214–215 °C.

Das Ausgangsmaterial wurde dadurch erhalten, dass man 1,85 g gemäss Beispiel 1 erhaltenen 1-Äthyl-6-(4-chlorphenyl)-4-oxo-1,4,5,6-tetrahydronicotinsäuremethylester in 10 ml Dioxan und 10 ml Methanol löste und in Gegenwart von 20 ml 1 N Natronlauge während 3 Stunden unter Rückfluss erhitzte. Man kühlte ab, säuerte mit 0,5 N Salzsäure an, nutschte die ausgefallenen Kristalle ab und kristallisierte sie aus Methanol/Äther um. Ausbeute 1,3 g (74%), Smp. 142–144 °C.

Beispiel 3

In Analogie zu den Beispielen 1 und 2 wurde

aus Benzaldehyd 2-(Äthylaminomethylen)-3-oxo-5-phenyl-4-pentensäuremethylester, Smp. 92–94 °C, hergestellt, dieser zu 1-Äthyl-4-oxo-6-phenyl-1,4,5,6-tetrahydronicotinsäure, Smp. 148–150 °C, cyclisiert und verseift und anschliessend zu 1-Äthyl-4-oxo-6-phenyl-1,4-dihydronicotinsäure, Smp. 166–167 °C, dehydriert.

Beispiel 4

In Analogie zu Beispiel 1 wurde aus 4-(Dimethylamino)-benzaldehyd 2-(Äthylaminomethylen)-5-[4-(dimethylamino)-phenyl]-3-oxo-4-pentensäuremethylester, Smp. 129–130 °C, hergestellt, dieser zu 1-Äthyl-6-[4-(dimethylamino)-phenyl]-4-oxo-1,4,5,6-tetrahydronicotinsäuremethylester, Smp. 85–87 °C, cyclisiert und zu 1-Äthyl-6-[4-(dimethylamino)-phenyl]-4-oxo-1,4-dihydronicotinsäure, Smp. 258–259 °C, dehydriert und verseift.

Beispiel 5

In Analogie zu Beispiel 1 wurde aus 3-Methyl-4-(dimethylamino)-benzaldehyd 2-(Äthylaminomethylen)-5-[3-methyl-4-(dimethylamino)-phenyl]-3-oxo-4-pentensäuremethylester, Smp. 98–100 °C, hergestellt, dieser zu 1-Äthyl-6-[3-methyl-4-(dimethylamino)-phenyl]-4-oxo-1,4,5,6-tetrahydronicotinsäuremethylester cyclisiert, zu 1-Äthyl-6-[3-methyl-4-(dimethylamino)-phenyl]-4-oxo-1,4-dihydronicotinsäuremethylester, Smp. 132–134 °C, dehydriert und zu 1-Äthyl-6-[3-methyl-4-(dimethylamino)-phenyl]-4-oxo-1,4-dihydronicotinsäure, Smp. 160–162 °C, verseift.

Beispiel 6

In Analogie zu den Beispielen 1 und 2 wurde aus 4-(1-Pyrrolyl)benzaldehyd 2-(Äthylaminomethylen)-3-oxo-5-[4-(1-pyrrolyl)phenyl]-4-pentensäuremethylester, Smp. 119–121 °C, hergestellt, dieser zu 1-Äthyl-6-[4-(1-pyrrolyl)phenyl]-4-oxo-1,4,5,6-tetrahydronicotinsäure cyclisiert und verseift und anschliessend zu 1-Äthyl-6-[4-(1-pyrrolyl)phenyl]-4-oxo-1,4-dihydronicotinsäure, Smp. >300 °C, dehydriert.

Beispiel 7

In Analogie zu den Beispielen 1 und 2 wurde aus 4-Methoxybenzaldehyd 2-(Äthylaminomethylen)-3-oxo-5-(4-methoxyphenyl)-4-pentensäuremethylester hergestellt, dieser zu 1-Äthyl-6-(4-methoxyphenyl)-4-oxo-1,4,5,6-tetrahydronicotinsäure, Smp. 173–174 °C, cyclisiert und verseift und anschliessend zu 1-Äthyl-6-(4-methoxyphenyl)-4-oxo-1,4-dihydronicotinsäure, Smp. 215–217 °C, dehydriert.

Beispiel 8

In Analogie zu den Beispielen 1 und 2 wurde aus 4-Methoxybenzaldehyd 2-(Cyclopropylaminomethylen)-5-(4-methoxyphenyl)-3-oxo-4-pentensäuremethylester, Smp. 125–127 °C, hergestellt, dieser zu 1-Cyclopropyl-6-(4-methoxyphenyl)-4-oxo-1,4,5,6-tetrahydronicotinsäure, Smp. 137–139 °C, cyclisiert und verseift und anschliessend zu 1-Cyclopropyl-6-(4-methoxyphenyl)-4-oxo-1,4-dihydronicotinsäure, Smp. 159–161 °C, dehydriert.

Beispiel 9

In Analogie zu den Beispielen 1 und 2 wurde aus 3,4-Dimethoxybenzaldehyd 2-(Äthylaminomethylen)-5-(3,4-dimethoxyphenyl)-3-oxo-4-pentensäuremethylester hergestellt, dieser zu 1-Äthyl-6-(3,4-dimethoxyphenyl)-4-oxo-1,4,5,6-tetrahydronicotinsäuremethylester, Smp. 134–135 °C, cyclisiert, zu 1-Äthyl-6-(3,4-dimethoxyphenyl)-4-oxo-1,4,5,6-tetrahydronicotinsäure, Smp. 182–183 °C, verseift und anschliessend zu 1-Äthyl-6-(3,4-dimethoxyphenyl)-4-oxo-1,4-dihydronicotinsäure, Smp. 232-234 °C, dehydriert.

Beispiel 10

In Analogie zu den Beispielen 1 und 2 wurde aus 3,4-Methylendioxybenzaldehyd 2-(Äthylaminomethylen)-5-(3,4-methylendioxyphenyl)-3-oxo-4-pentensäuremethylester, Smp. 124–125 °C, hergestellt, dieser zu 1-Äthyl-6-(3,4-methylendioxyphenyl)-4-oxo-1,4,5,6-tetrahydronicotinsäure, Smp. 173–175 °C, cyclisiert und verseift und anschliessend zu 1-Äthyl-6-(3,4-methylendioxyphenyl)-4-oxo-1,4-dihydronicotinsäure, Smp. 269–270 °C, dehydriert.

Beispiel 11

In Analogie zu den Beispielen 1 und 2 wurde aus 3,4-Methylendioxybenzaldehyd 2-(Cyclopropylaminomethylen)-5-(3,4-methylendioxyphenyl)-3-oxo-4-pentensäuremethylester, Smp. 158–160 °C, hergestellt, dieser zu 1-Cyclopropyl-6-(3,4-methylendioxyphenyl)-4-oxo-1,4,5,6-tetrahydronicotinsäure, Smp. 122–124 °C, cyclisiert und verseift und anschliessend zu 1-Cyclopropyl-6-(3,4-methylendioxyphenyl)-4-oxo-1,4-dihydronicotinsäure, Smp. 211–121 °C, dehydriert.

Beispiel 12

In Analogie zu den Beispielen 1 und 2 wurde aus 3,4,5-Trimethoxybenzaldehyd 2-(Äthylaminomethylen)-3-oxo-5-(3,4,5-trimethoxyphenyl)-4-pentensäuremethylester in Form eines Öls hergestellt, dieser zu 1-Äthyl-4-oxo-6-(3,4,5-timethoxyphenyl)-1,4,5,6-tetrahydronicotinsäuremethylester cyclisiert und anschliessend zu 1-Äthyl-4-oxo-6-(3,4,5-trimethoxyphenyl)-1,4-dihydronicotinsäure, Smp. 200–201 °C, dehydriert und verseift.

Beispiel 13

In Analogie zu den Beispielen 1 und 2 wurde aus 4-(Methylthio)-benzaldehyd 2-(Äthylaminomethylen)-5-(4-methylthiophenyl)-3-oxo-4-pentensäuremethylester hergestellt, dieser zu 1-Äthyl-6-(4-methylthiophenyl)-4-oxo-1,4,5,6-tetrahydronicotinsäure, Smp. 173–174 °C, cyclisiert und verseift und anschliessend zu 1-Äthyl-6-(4-methylthiophenyl)-4-oxo-1,4-dihydronicotinsäure, Smp. 195–197 °C, dehydriert.

Beispiel 14

In Analogie zu den Beispielen 1 und 2 wurde aus p-Hydroxybenzaldehyd 2-(Äthylaminomethylen)-5-(4-hydroxyphenyl)-3-oxo-4-pentensäuremethylester hergestellt, dieser zu 1-Äthyl-6-(4-hydroxyphenyl)-4-oxo-1,4,5,6-tetrahydronicotinsäure cyclisiert und verseift und anschliessend zu 1-Äthyl-6-(4-hydroxyphenyl)-4-oxo-1,4-dihydronicotinsäure, Smp. 243-245°C, dehydriert.

Diese Verbindung kann auch folgendermassen erhalten werden:

3,7 g einer 50%igen Natriumhydriddispersion werden in 50 ml Dimethylformamid (DMF) bei Raumtemperatur unter Rühren mit 5 g Äthylmercaptan versetzt. Anschliessend wird dazu die Lösung von 4,3 g 1-Äthyl-6-(4-methoxyphenyl)-4-oxo-1,4-dihydronicotinsäure (Beispiel 7) in 50 ml DMF zugefügt und das Gemisch auf 100°C erhitzt, wobei zunächst eine klare Lösung entsteht. Nach ca. 30 Minuten entsteht wieder ein Niederschlag. Man lässt 16 Stunden bei 100°C rühren, kühlt dann ab und nimmt das Reaktionsgemisch in Essigester auf. Diese Lösung wird mit kalter 0,1 N Salzsäure neutralgewaschen, mit Natriumsulfat getrocknet und das Lösungsmittel entfernt. Der Rückstand wird aus Methanol/Äther kristallisiert. Ausbeute 3,4 g (83%), Smp. 245-247°C.

Beispiel 15

In Analogie zu den Beispielen 1 und 2 wurde aus m-Chlorbenzaldehyd 2-(Äthylaminomethylen)-5-(3-chlorphenyl)-3-oxo-4-pentensäuremethylester hergestellt, dieser zu 1-Äthyl-6-(3-chlorphenyl)-4-oxo-1,4,5,6-tetrahydronicotinsäure, Smp. 143-145°C, cyclisiert und verseift und anschliessend zu 1-Äthyl-6-(3-chlorphenyl)-4-oxo-1,4-dihydronicotinsäure, Smp. 212-213°C, dehydriert.

Beispiel 16

In Analogie zu den Beispielen 1 und 2 wurde aus 2,6-Dichlorbenzaldehyd 2-(Äthylaminomethylen)-5-(2,6-dichlorphenyl)-3-oxo-4-pentensäuremethylester hergestellt, dieser zu 1-Äthyl-6-(2,6-dichlorphenyl)-4-oxo-1,4,5,6-tetrahydronicotinsäure cyclisiert und verseift und anschliessend zu 1-Äthyl-6-(2,6-dichlorphenyl)-4-oxo-1,4-dihydronicotinsäure, Smp. 200-201°C, dehydriert.

Beispiel 17

In Analogie zu den Beispielen 1 und 2 wurde aus p-Chlorbenzaldehyd 5-(4-Chlorphenyl)-2-(methylaminomethylen)-3-oxo-4-pentensäuremethylester, Smp. 114-116°C, hergestellt, dieser zu 6-(4-Chlorphenyl)-1-methyl-4-oxo-1,4,5,6-tetrahydronicotinsäure, Smp. 205-206°C, cyclisiert und verseift und anschliessend zu 6-(4-Chlorphenyl)-1-methyl-4-oxo-1,4-dihydronicotinsäure, Smp. 275-280°C (Zers.), dehydriert.

Beispiel 18

In Analogie zu den Beispielen 1 und 2 wurde aus 3-Methyl-4-methylamino-benzaldehyd 2-(Äthylaminomethylen)-5-(3-methyl-4-methylaminophenyl)-3-oxo-4-pentensäuremethylester hergestellt, dieser zu 1-Äthyl-6-(3-methyl-4-methylaminophenyl)-4-oxo-1,4,5,6-tetrahydronicotinsäure cyclisiert und verseift und anschliessend zu 1-Äthyl-6-(3-methyl-4-methylaminophenyl)-4-oxo-1,4-dihydronicotinsäure, Smp. 243-244°C, dehydriert.

Beispiel 19

In Analogie zu den Beispielen 1 und 2 wurde aus Nicotinaldehyd 2-(Äthylaminomethylen)-3-oxo-5-(3-pyridyl)-4-pentensäuremethylester, Smp. 78-80°C, hergestellt, dieser zu 1-Äthyl-4-oxo-6-(3-pyridyl)-1,4,5,6-tetrahydronicotinsäure, Smp.197-199°C, cyclisiert und verseift und anschliessend zu 1-Äthyl-4-oxo-6-(3-pyridyl)-1,4-dihydronicotinsäure, Smp. 209-210°C, dehydriert.

In analoger Weise wurde aus Isonicotinaldehyd 1-Äthyl-4-oxo-6-(4-pyridyl)-1,4-dihydronicotinsäure, Smp. 268-270°C, hergestellt.

Beispiel 20

In Analogie zu den Beispielen 1 und 2 wurde aus 4-(1-Pyrrolidinyl)-benzaldehyd 2-(Äthylaminomethylen)-3-oxo-5-[4-(1-pyrrolidinyl)phenyl]-4-pentensäuremethylester hergestellt, dieser zu 1-Äthyl-4-oxo-6-[4-(1-pyrrolidinyl)phenyl]-1,4,5,6-tetrahydronicotinsäure cyclisiert und verseift und anschliessend zu 1-Äthyl-6-[4-(1-pyrrolidinyl)phenyl]-4-oxo-1,4-dihydronicotinsäure, Smp. 276-278°C, dehydriert.

Diese Verbindung kann auch folgendermassen erhalten werden:

7,8 g 1-Äthyl-4-oxo-6-[4-(1-pyrrolyl)phenyl]-1,4-dihydronicotinsäuremethylester werden bei Raumtemperatur mit 8,5 g 5% Palladiumkohle in 800 ml Methanol hydriert. Nach Entfernung des Katalysators wird das Reaktionsgemisch zur Trockene eingedampft und der Rückstand mit Methylenchlorid aufgenommen. Die organische Lösung wird mit eiskalter 1 N Salzsäure extrahiert, der wässrige Extrakt mit 1 N Natronlauge neutralisiert und mit Methylenchlorid extrahiert. Nach Trocknen der Lösung und Eindampfen erhält man 7,0 g 1-Äthyl-4-oxo-6-[4-(1-pyrrolidinyl)phenyl]-1,4-dihydronicotinsäuremethylester, Smp. 157-159°C (aus Methanol).

In Analogie zu Beispiel 1 kann der Ester dann zur Säure verseift werden.

Beispiel 21

1,25 g 1-Äthyl-6-(3-nitrophenyl)-4-oxo-1,4-dihydronicotinsäure in 60 ml Dimethylformamid wurden in Gegenwart von 100 mg Palladiumkohle (5%, w/w) 50 Minuten bei Raumtemperatur hydriert. Der Katalysator wurde abfiltriert, das Lösungsmittel entfernt und der Rückstand aus Äthanol/Äther kristallisiert. Es wurden 900 mg (80%) 1-Äthyl-6-(3-aminophenyl)-4-oxo-1,4-dihydronicotinsäure, Smp. 145-146,5°C, erhalten.

Beispiel 22

Es wurden Tabletten zu 120 mg bzw. 500 mg hergestellt, enthaltend

| | | |
|---|---|---|
| 1-Äthyl-4-oxo-6-phenyl-1,4-dihydronicotinsäure | 10,0 mg | - |
| 1-Äthyl-6-<4-(dimethylamino)-phenyl>-4-oxo-1,4-dihydronicotinsäure | - | 150 mg |
| Maisstärke | 50,0 mg | 160 mg |
| Milchzucker | 58,0 mg | 180 mg |
| Talk | 1,5 mg | 7 mg |
| Magnesiumstearat | 0,5 mg | 3 mg |
| | 120,0 mg | 500 mg |

## Patentansprüche

1. 4-Pyridon-4-carbonsäurederivate der allgemeinen Formel

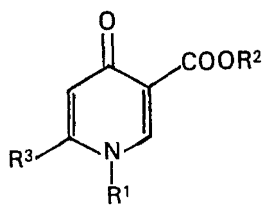

I

worin
$R^1$ $C_{1-8}$-Alkyl, $C_{3-10}$-Cycloalkyl, $C_{3-10}$-Cycloalkyl-$C_{1-6}$-alkyl, $C_{1-6}$-Alkoxy oder $C_{1-6}$-Alkoxy-$C_{1-6}$-alkyl;
$R^2$ Wasserstoff;
$R^3$ einen ggf. durch $R^4$, $R^5$ und/oder $R^6$ substituierten Phenyl-, Phenäthyl- oder Styrylrest oder einen ggf. durch $R^7$ substituierten, ein oder mehrere N-Atome enthaltenden aromatisch heterocyclischen 6-Ring, der über ein Ring-C-atom gebunden ist;
$R^4$ und $R^5$ Halogen, Trifluormethyl, Hydroxy, $C_{1-6}$-Alkoxy, $C_{1-6}$-Alkylthio, $C_{1-6}$-Alkyl, Amino, Acylamino, $C_{1-6}$Alkylamino, Di-$C_{1-6}$-alkylamino, Nitro, einen über ein Ring-N- oder -C-atom gebundenen 5- oder 6-gliedrigen Heterocyclus oder beide Reste $R^4$ und $R^5$ gemeinsam $C_{1-6}$-Alkylendioxy;
$R^6$ $C_{1-6}$-Alkoxy und
$R^7$ Halogen, Hydroxy, $C_{1-6}$-Alkoxy, $C_{1-6}$-Alkylthio, $C_{1-6}$-Alkyl, Amino, $C_{1-6}$-Alkylamino oder Di-$C_{1-6}$-alkylamino bedeuten,
- mit Ausnahme von 1-Methyl-4-oxo-6-phenyl-1,4-dihydronicotinsäure und deren Salze.

2. 4-Pyridon-3-carbonsäurederivate der allgemeinen Formel

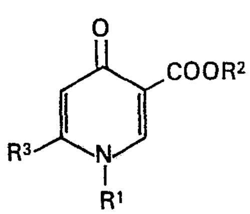

I

worin
$R^1$ $C_{1-6}$-Alkyl oder $C_{3-6}$-Cycloalkyl;
$R^3$ einen ggf. durch $R^4$, $R^5$ und/oder $R^6$ substituierten Phenylrest oder einen ggf. durch $R^7$ substituierten, ein oder mehrere N-Atome enthaltenden aromatisch heterocyclischen 6-Ring, der über ein Ring-C-atom gebunden ist;
$R^4$ und $R^5$ Halogen, Hydroxy, $C_{1-6}$-Alkoxy, $C_{1-6}$-Alkylthio, $C_{1-6}$-Alkyl, Amino, $C_{1-6}$-Alkylamino, Di-$C_{1-6}$-alkylamino, einen über ein Ring-N- oder -C-atom gebundenen 5- oder 6-gliedrigen Heterocyclus oder beide Reste $R^4$ und $R^5$ gemeinsam $C_{1-6}$-Alkylendioxy bedeuten
und $R^2$, $R^6$ und $R^7$ die in Anspruch 1 angegebenen Bedeutungen besitzen - mit Ausnahme von 1-Methyl-4-oxo-6-phenyl-1,4-dihydronicotinsäure und deren Salze.

3. eine Verbindung gemäss Anspruch 1 oder Anspruch 2, dadurch gekennzeichnet, dass $R^1$ Methyl oder Äthyl ist.

4. Eine Verbindung gemäss einem der Ansprüche 1-4, dadurch gekennzeichnet, dass $R^3$ ein substituierter Phenylrest ist.

5. Eine Verbindung aus der Gruppe der folgenden:

1-Äthyl-6-(4-chlorphenyl)-4-oxo-1,4-dihydronicotinsäure;
1-Äthyl-4-oxo-6-phenyl-1,4-dihydronicotinsäure;
1-Äthyl-6-[4-(dimethylamino)-phenyl]-4-oxo-1,4-dihydronicotinsäure;
1-Äthyl-6-[3-methyl-4-(dimethylamino)-phenyl]-4-oxo-1,4-dihydronicotinsäure;
1-Äthyl-6-[4-(1-pyrrolyl)phenyl]-4-oxo-1,4-dihydronicotinsäure;
1-Äthyl-6-(4-methoxyphenyl)-4-oxo-1,4-dihydronicotinsäure;
1-Cyclopropyl-6-(4-methoxyphenyl)-4-oxo-1,4-dihydronicotinsäure;
1-Äthyl-6-(3,4-dimethoxyphenyl)-4-oxo-1,4-dihydronicotinsäure;
1-Äthyl-6-(3,4-methylendioxyphenyl)-4-oxo-1,4-dihydronicotinsäure;
1-Cyclopropyl-6-(3,4-methylendioxyphenyl)-4-oxo-1,4-dihydronicotinsäure;
1-Äthyl-4-oxo-6-(3,4,5-trimethoxyphenyl)-1,4-dihydronicotinsäure;
1-Äthyl-6-(4-methylthiophenyl)-4-oxo-1,4-dihydronicotinsäure;
1-Äthyl-6-(4-hydroxyphenyl)-4-oxo-1,4-dihydronicotinsäure;
1-Äthyl-6-(3-chlorphenyl)-4-oxo-1,4-dihydronicotinsäure;
1-Äthyl-6-(2,6-dichlorphenyl)-4-oxo-1,4-dihydronicotinsäure;

6-(4-Chlorphenyl)-1-methyl-4-oxo-1,4-dihydronicotinsäure;

1-Äthyl-6-(3-methyl-4-methylaminophenyl)-4-oxo-1,4-dihydronicotinsäure;

1-Äthyl-4-oxo-6-(3-pyridyl)-1,4-dihydronicotinsäure;

1-Äthyl-6-[4-(1-pyrrolidinyl)phenyl]-4-oxo-1,4-dihydronicotinsäure;

6. Eine Verbindung aus der Gruppe der folgenden:

4-Oxo-6-phenyl-1-propyl-1,4-dihydronicotinsäure;

1-Isopropyl-4-oxo-6-phenyl-1,4-dihydronicotinsäure;

1-Butyl-4-oxo-6-phenyl-1,4-dihydronicotinsäure;

1-tert.-Butyl-4-oxo-6-phenyl-1,4-dihydronicotinsäure;

1-Octyl-4-oxo-6-phenyl-1,4-dihydronicotinsäure;

1-(Cyclopropylmethyl)-4-oxo-6-phenyl-1,4-dihydronicotinsäure;

1-Cyclopropyl-4-oxo-6-phenyl-1,4-dihydronicotinsäure;

1-Cyclopentyl-4-oxo-6-phenyl-1,4-dihydronicotinsäure;

1-Cyclohexyl-4-oxo-6-phenyl-1,4-dihydronicotinsäure;

1-(1-Adamantyl)-4-oxo-6-phenyl-1,4-dihydronicotinsäure;

1-(2-Methoxyäthyl)-4-oxo-6-phenyl-1,4-dihydronicotinsäure;

1-Methoxy-4-oxo-6-phenyl-1,4-dihydronicotinsäure;

1-Butyloxy-4-oxo-6-phenyl-1,4-dihydronicotinsäure;

1-Äthyl-6-(3-nitrophenyl)-4-oxo-1,4-dihydronicotinsäure;

1-Äthyl-6-(3-aminophenyl)-4-oxo-1,4-dihydronicotinsäure;

6-[4-(Dimethylamino)-phenyl]-1-methoxy-4-oxo-1,4-dihydronicotinsäure;

1-Butoxy-6-[4-(dimethylamino)-phenyl]-4-oxo-1,4-dihydronicotinsäure;

6-(4-Acetamidophenyl)-1-äthyl-4-oxo-1,4-dihydronicotinsäure;

1-Äthyl-4-oxo-6-[3-trifluormethyl)-phenyl]-1,4-dihydronicotinsäure;

1-Äthyl-6-[4-(4-methyl-1-piperazinyl)-phenyl]-4-oxo-1,4-dihydronicotinsäure;

1-Äthyl-6-(p-methoxystyryl)-4-oxo-1,4-dihydronicotinsäure;

1-Äthyl-4-oxo-6-(4-pyridyl)-1,4-dihydronicotinsäure

7. Verfahren zur Herstellung von 4-Pyridon-3-carbonsäurederivaten gemäss Anspruch 1, dadurch gekennzeichnet, dass man

(a) ein 1,4,5,6-Tetrahydronicotinsäurederivat der Formel

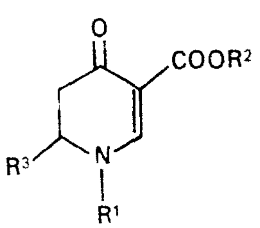

II

worin $R^1$, $R^2$ und $R^3$ die in Anspruch 1 angegebenen Bedeutungen besitzen, in 5,6-Stellung dehydriert oder

(b) einen Substituenten $R^3$ innerhalb der oben angegebenen Definitionen in an sich bekannter Weise modifiziert

und gewünschtenfalls die erhaltene Verbindung der Formel I in ein Salz überführt.

8. Verfahren zur Herstellung von 4-Pyridon-3-carbonsäurederivaten gemäss Anspruch 2, dadurch gekennzeichnet, dass man

(a) ein 1,4,5,6-Tetrahydronicotinsäurederivat der Formel

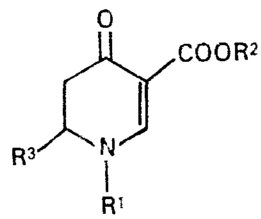

II

worin $R^1$, $R^2$ und $R^3$ die in Anspruch 2 angegebenen Bedeutungen besitzen,

in 5,6-Stellung dehydriert oder

(b) einen Substituenten $R^3$ innerhalb der oben angegebenen Definitionen in an sich bekannter Weise modifiziert

und gewünschtenfalls die erhaltene Verbindung der Formel I in ein Salz überführt.

9. Arzneimittel auf der Basis eines 4-Pyridon-3-carbonsäurederivats gemäss einem der Ansprüche 1–6.

10. Antibakterielle Mittel auf der Basis eines 4-Pyridon-3-carbonsäurederivats gemäss einem der Ansprüche 1–6.

11. Zentralnervensystem-stimulierende Mittel auf der Basis eines 4-Pyridon-3-carbonsäurederivats gemäss einem der Ansprüche 1-6.

12. 4-Pyridon-3-carbonsäurederivate gemäss einem der Ansprüche 1–6 zur Anwendung als pharmazeutische Wirkstoffe.

13. 4-Pyridon-3-carbonsäurederivate gemass einem der Ansprüche 1–6 zur Anwendung als antibakterielle Wirkstoffe.

14. 4-Pyridon-3-carbonsäurederivate gemass einem der Ansprüche 1–6 zur Anwendung als Stoffe mit stimulierender Wirkung auf das Zentralnervensystem.

## Revendications

1. Dérivés d'acide 4-pyridone-3-carboxylique de formule générale

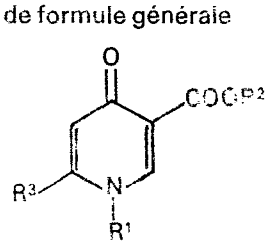

dans laquelle $R^1$ représente un groupe alkyle en $C_1$–$C_8$, un groupe cycloalkyle en $C_3$–$C_6$, un groupe cycloalkylalkyle à reste cycloalkyle en $C_3$–$C_6$ et

à reste alkyle en $C_1$–$C_6$, un groupe alcoxy en $C_1$–$C_6$ ou un groupe alcoxyalkyle à reste alcoxy en $C_1$–$C_6$ et à reste alkyle en $C_1$–$C_6$, $R^2$ représente de l'hydrogène, $R^3$ représente un reste phényle, phénéthyle ou styryle éventuellement substitué par des restes $R^4$, $R^5$ et/ou $R^6$ ou un noyau hexagonal aromatique hétérocyclique éventuellement substitué par un groupe $R^7$ et contenant un ou plusieurs atomes d'azote et qui est fixé par l'intermédiaire d'un atome de carbone du cycle, $R^4$ et $R^5$ représentent un halogène, un groupe trifluorométhyle, un groupe hydroxy, un groupe alcoxy en $C_1$–$C_6$, un groupe alkylthio en $C_1$–$C_6$, un groupe alkyle en $C_1$–$C_6$, un groupe amino, un groupe acylamino, un groupe alkylamino en $C_1$–$C_6$, un groupe dialkylamino à restes alkyle en $C_1$–$C_6$, un groupe nitro ou un hétérocycle à 5 ou 6 maillons, fixé par l'intermédiaire d'un atome d'azote ou de carbone du cycle, ou encore, les deux restes $R^4$ et $R^5$ représentent ensemble un groupe alkylènedioxy en $C_1$–$C_6$, $R^6$ représente un groupe alcoxy en $C_1$–$C_6$ et $R^7$ représente un halogène, un groupe hydroxy, un groupe alcoxy en $C_1$–$C_6$, un groupe alkylthio en $C_1$–$C_6$, un groupe alkyle en $C_1$–$C_6$, un groupe amino, un groupe alkylamino en $C_1$–$C_6$ ou un groupe dialkylamino à restes alkyle en $C_1$–$C_6$, à l'exception de l'acide 1-méthyl-4-oxo-6-phényl-1,4-dihydronicotinique, et leurs sels.

2. Dérivés d'acide 4-pyridone-3-carboxylique de formule générale

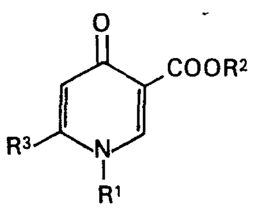

I

dans laquelle $R^1$ représente un groupe alkyle en $C_1$–$C_6$ ou un groupe cycloalkyle en $C_3$–$C_6$, $R^3$ représente un reste phényle éventuellement substitué par des groupes $R^4$, $R^5$ et/ou $R^6$ ou un noyau hexagonal aromatiquement hétérocyclique éventuellement substitué par un groupe $R^7$ et contenant un ou plusieurs atomes d'azote et qui est fixé par l'intermédiaire d'un atome de carbone du cycle, $R^4$ et $R^5$ représentent un halogène, un groupe hydroxy, un groupe alcoxy en $C_1$–$C_6$, un groupe alkylthio en $C_1$–$C_6$, un groupe alkyle en $C_1$–$C_6$, un groupe amino, un groupe alkylamino en $C_1$–$C_6$, un groupe dialkylamino à restes alkyle en $C_1$–$C_6$ ou un hétérocycle à 5 ou 6 maillons, fixé par l'intermédiaire d'un atome d'azote ou de carbone du cycle, ou encore, les deux restes $R^4$ et $R^5$ représentent ensemble un groupe alkylènedioxy en $C_1$–$C_6$, et $R^2$, $R^6$ et $R^7$ ont la même signification que dans la revendication 1, à l'exception de l'acide 1-méthyl-4-oxo-6-phényl-1,4-dihydronicotinique, et leurs sels.

3. Composé suivant la revendication 1 ou la revendication 2, caractérisé par le fait que $R^1$ représente un groupe méthyle ou éthyle.

4. Composé suivant l'une quelconque des revendications 1 à 3, caractérisé par le fait que $R^3$ représente un reste phényle substitué.

5. Composé du groupe comprenant:

- l'acide 1-éthyl-6-(4-chlorophényl)-4-oxo-1,4-dihydronicotinique,
- l'acide 1-éthyl-4-oxo-6-phényl-1,4-dihydronicotinique,
- l'acide 1-éthyl-6-[4-(diméthylamino)-phényl]-4-oxo-1,4-dihydronicotinique,
- l'acide 1-éthyl-6-[3-méthyl-4-(diméthylamino)-phényl]-4-oxo-1,4-dihydronicotinique,
- l'acide 1-éthyl-6-[4-(1-pyrrolyl)-phényl]-4-oxo-1,4-dihydronicotinique,
- l'acide 1-éthyl-6-(4-méthoxyphényl)-4-oxo-1,4-dihydronicotinique,
- l'acide 1-cyclopropyl-6-(4-méthoxyphényl)-4-oxo-1,4-dihydronicotinique,
- l'acide 1-éthyl-6-(3,4-diméthoxyphényl)-4-oxo-1,4-dihydronicotinique,
- l'acide 1-éthyl-6-(3,4-méthylènedioxyphényl)-4-oxo-1,4-dihydronicotinique,
- l'acide 1-cyclopropyl-6-(3,4-méthylènedioxyphényl)-4-oxo-1,4-dihydronicotinique,
- l'acide 1-éthyl-4-oxo-6-(3,4,5-triméthoxyphényl)-1,4-dihydronicotinique,
- l'acide 1-éthyl-6-(4-méthylthiophényl)-4-oxo-1,4-dihydronicotinique,
- l'acide 1-éthyl-6-(4-hydroxyphényl)-4-oxo-1,4-dihydronicotinique,
- l'acide 1-éthyl-6-(3-chlorphényl)-4-oxo-1,4-dihydronicotinique,
- l'acide 1-éthyl-6-(2,6-dichlorophényl)-4-oxo-1,4-dihydronicotinique,
- l'acide 6-(4-chlorophényl)-1-méthyl-4-oxo-1,4-dihydronicotinique,
- l'acide 1-éthyl-6-(3-méthyl-4-méthylaminophényl)-4-oxo-1,4-dihydronicotinique,
- l'acide 1-éthyl-4-oxo-6-(3-pyridyl)-1,4-dihydronicotinique,
- l'acide 1-éthyl-6-[4-(1-pyrrolidinyl)-phényl]-4-oxo-1,4-dihydronicotinique.

6. Composé du groupe comprenant:

- l'acide 4-oxo-6-phényl-1-propyl-1,4-dihydronicotinique,
- l'acide 1-isopropyl-4-oxo-6-phényl-1,4-dihydronicotinique,
- l'acide 1-butyl-4-oxo-6-phényl-1,4-dihydronicotinique,
- l'acide 1-tert.-butyl-4-oxo-6-phényl-1,4-dihydronicotinique,
- l'acide 1-octyl-4-oxo-6-phényl-1,4-dihydronicotinique,
- l'acide 1-(cyclopropylméthyl)-4-oxo-6-phényl-1,4-dihydronicotinique,
- l'acide 1-cyclopropyl-4-oxo-6-phényl-1,4-dihydronicotinique,
- l'acide 1-cyclopentyl-4-oxo-6-phényl-1,4-dihydronicotinique,
- l'acide 1-cyclohexyl-4-oxo-6-phényl-1,4-dihydronicotinique,
- l'acide 1-(1-adamantyl)-4-oxo-6-phényl-1,4-dihydronicotinique,
- l'acide 1-(2-méthoxyéthyl)-4-oxo-6-phényl-1,4-dihydronicotinique,

- l'acide 1-méthoxy-4-oxo-6-phényl-1,4-dihydronicotinique,
- l'acide 1-butyloxy-4-oxo-6-phényl-1,4-dihydronicotinique,
- l'acide 1-éthyl-6-(3-nitrophényl)-4-oxo-1,4-dihydronicotinique,
- l'acide 1-éthyl-6-(3-aminophényl)-4-oxo-1,4-dihydronicotinique,
- l'acide 6-[4-(diméthylamino)-phényl]-1-méthoxy-4-oxo-1,4-dihydronicotinique,
- l'acide 1-butoxy-6-[-(diméthylamino)-phényl]-4-oxo-1,4-dihydronicotinique,
- l'acide 6-(4-acétamidophényl)-1-éthyl-4-oxo-1,4-dihydronicotinique,
- l'acide 1-éthyl-4-oxo-6-[3-(trifluorométhyl)-phényl]-1,4-dihydronicotinique,
- l'acide 1-éthyl-6-[4-(4-méthyl-1-pipérazinyl)-phényl]-4-oxo-1,4-dihydronicotinique,
- l'acide 1-éthyl-6-(p-méthoxystyryl)-4-oxo-1,4-dihydronicotinique,
- l'acide 1-éthyl-4-oxo-6-(4-pyridyl)-1,4-dihydronicotinique.

7. Procédé pour la préparation des dérivés d'acide 4-pyridone-3-carboxylique selon la revendication 1, procédé caractérisé par le fait que a) on déshydrogène en position 5,6 un dérivé d'acide 1,4,5,6-tétrahydronicotinique de formule

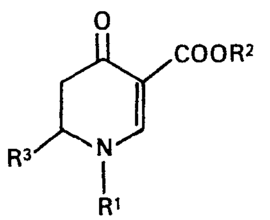

II

dans laquelle $R^1$, $R^2$ et $R^3$ ont les significations indiquées dans la revendication 1, ou que
b) on modifie, de manière connue en soi, le substituant $R^3$ dans le cadre des définitions précitées et, si on le désire, on transforme en un sel le composé de formule I ainsi obtenu.

8. Procédé pour la préparation des dérivés d'acide 4-pyridone-3-carboxylique selon la revendication 2, procédé caractérisé par le fait que a) on déshydrogène en position 5,6 un dérivé d'acide 1,4,5,6-tétrahydronicotinique de formule

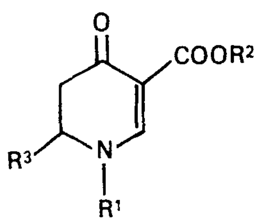

II

dans laquelle $R^1$,$R^2$ et $R^3$ ont les significations indiquées dans la revendication 2, ou que
b) on modifie, de manière connue en soi, le substituant $R^3$ dans le cadre des définitions ci-dessus et, si on le désire, on transforme en un sel le composé de formule I ainsi obtenu.

9. Médicaments à base d'un dérivé d'acide 4-pyridone-3-carboxylique suivant l'une des revendications 1 à 6.

10. Agents antibactériens à base d'un dérivé d'acide 4-pyridone-3-carboxylique suivant l'une des revendications 1 à 6.

11. Agents exerçant une action stimulante sur le système nerveux central, à base d'un dérivé d'acide 4-pyridone-3-carboxylique suivant l'une des revendications 1 à 6.

12. Dérivés d'acide 4-pyridone-3-carboxylique suivant l'une des revendications 1 à 6 pour l'utilisation comme agents pharmaceutiques.

13. Dérivés d'acide 4-pyridone-3-carboxylique suivant l'une des revendications 1 à 6 pour l'utilisation comme agents antibactériens.

14. Dérivés d'acide 4-pyridone-3-carboxylique suivant l'une des revendications 1 à 6 pour l'utilisation comme agents stimulant le système nerveux central.

**Claims**

1. Derivatives of 4-pyridone-3-carboxylic acid of the general formula

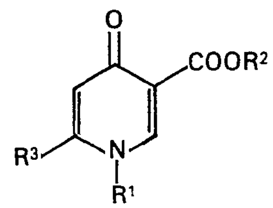

I

wherein
$R^1$ represents a $C_{1-8}$-alkyl, $C_{3-10}$-cycloalkyl, $C_{3-10}$-cycloalkyl-$C_{1-6}$-alkyl, $C_{1-6}$-alkoxy or $C_{1-6}$-alkoxy-$C_{1-6}$-alkyl group;
$R^2$ represents a hydrogen atom;
$R^3$ represents a phenyl, phenethyl or styryl group, which is optionally substituted by $R^4$, $R^5$ and/or $R^6$, or an aromatic heterocyclic 6-membered ring containing one or more N-atoms, which is linked via a ring C-atom and which is optionally substituted by $R^7$;
$R^4$ and $R^5$ each represent a halogen atom or a trifluoromethyl, hydroxy, $C_{1-6}$-alkoxy, $C_{1-6}$-alkylthio, $C_{1-6}$-alkyl, amino, acylamino, $C_{1-6}$-alkylamino, di-$C_{1-6}$-alkylamino or nitro group or a 5-membered or 6-membered heterocycle linked via a ring N- or C-atom or $R^4$ and $R^5$ together represent a $C_{1-6}$-alkylenedioxy group;
$R^6$ represents a $C_{1-6}$-alkoxy group and
$R^7$ represents a halogen atom or a hydroxy, $C_{1-6}$-alkoxy, $C_{1-6}$-alkylthio, $C_{1-6}$-alkyl, amino, $C_{1-6}$-alkylamino or di-$C_{1-6}$-alkylamino group;
with the exception of 1-methyl-4-oxo-6-phenyl-1,4-dihydronicotinic acid and salts thereof.

2. Derivatives of 4-pyridone-3-carboxylic acid of the general formula

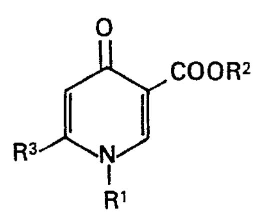

I

wherein $R^1$ represents a $C_{1-6}$-alkyl or $C_{3-6}$-cycloalkyl group;
$R^3$ represents a phenyl group, which is optionally substituted by $R^4$, $R^5$ and/or $R^6$, or an aromatic heterocyclic 6-membered ring containing one or more N-atoms, which is linked via a ring C-atom and which is optionally substituted by $R^7$;
$R^4$ and $R^5$ each represent a halogen atom or a hydroxy, $C_{1-6}$-alkoxy, $C_{1-6}$-alkylthio, $C_{1-6}$-alkyl, amino, $C_{1-6}$-alkylamino or di-$C_{1-6}$-alkylamino group or a 5-membered or 6-membered heterocycle linked via a ring N- or C-atom or $R^4$ and $R^5$ together represent a $C_{1-6}$-alkylenedioxy group; and wherein $R^2$, $R^6$ and $R^7$ are as defined in claim 1, with the exception of 1-methyl-4-oxo-6-phenyl-1,4-dihydronicotinic acid and salts thereof.

3. A compound according to claim 1 or claim 2, wherein $R^1$ represents the methyl or ethyl group.

4. A compound according to any one of claims 1 to 3, wherein $R^3$ represents a substituted phenyl group.

5. A compound selected from the following group:
1-ethyl-6-(4-chlorphenyl)-4-oxo-1,4-dihydronicotinic acid;
1-ethyl-4-oxo-6-phenyl-1,4-dihydronicotinic acid;
1-ethyl-6-[4-(dimethylamino)-phenyl]-4-oxo-1,4-dihydronicotinic acid;
1-ethyl-6-[3-methyl-4-(dimethylamino)-phenyl]-4-oxo-1,4-dihydronicotinic acid;
1-ethyl-6-[4-(1-pyrrolyl)-phenyl]-4-oxo-1,4-dihydronicotinic acid;
1-ethyl-6-(4-methoxyphenyl)-4-oxo-1,4-dihydronicotinic acid;
1-cyclopropyl-6-(4-methoxyphenyl)-4-oxo-1,4-dihydronicotinic acid;
1-ethyl-6-(3,4-dimethoxyphenyl)-4-oxo-1,4-dihydronicotinic acid;
1-ethyl-6-(3,4-methylenedioxyphenyl)-4-oxo-1,4-dihydronicotinic acid;
1-cyclopropyl-6-(3,4-methylenedioxyphenyl)-4-oxo-1,4-dihydronicotinic acid;
1-ethyl-4-oxo-6-(3,4,5-trimethoxyphenyl)-1,4-dihydronicotinic acid;
1-ethyl-6-(4-methylthiophenyl)-4-oxo-1,4-dihydronicotinic acid;
1-ethyl-6-(4-hydroxyphenyl)-4-oxo-1,4-dihydronicotinic acid;
1-ethyl-6-(3-chlorphenyl)-4-oxo-1,4-dihydronicotinic acid;
1-ethyl-6-(2,6-dichlorophenyl)-4-oxo-1,4-dihydronicotinic acid;
6-(4-chlorophenyl)-1-methyl-4-oxo-1,4-dihydronicotinic acid;
1-ethyl-6-(3-methyl-4-methylaminophenyl)-4-oxo-1,4-dihydronicotinic acid;
1-ethyl-4-oxo-6-(3-pyridyl)-1,4-dihydronicotinic acid;
1-ethyl-6-[4-(1-pyrrolidinyl)-phenyl]-4-oxo-1,4-dihydronicotinic acid.

6. A compound selected from the following group:
4-oxo-6-phenyl-1-propyl-1,4-dihydronicotinic acid;
1-isopropyl-4-oxo-6-phenyl-1,4-dihydronicotinic acid;
1-butyl-4-oxo-6-phenyl-1,4-dihydronicotinic acid;
1-tert.-butyl-4-oxo-6-phenyl-1,4-dihydronicotinic acid;
1-octyl-4-oxo-6-phenyl-1,4-dihydronicotinic acid;
1-(cyclopropylmethyl)-4-oxo-6-phenyl-1,4-dihydronicotinic acid;
1-cyclopropyl-4-oxo-6-phenyl-1,4-dihydronicotinic acid;
1-cyclopentyl-4-oxo-6-phenyl-1,4-dihydronicotinic acid;
1-cyclohexyl-4-oxo-6-phenyl-1,4-dihydronicotinic acid;
1-(1-adamantyl)-4-oxo-6-phenyl-1,4-dihydronicotinic acid;
1-(2-methoxyethyl)-4-oxo-6-phenyl-1,4-dihydronicotinic acid;
1-methoxy-4-oxo-6-phenyl-1,4-dihydronicotinic acid;
1-butoxy-4-oxo-6-phenyl-1,4-dihydronicotinic acid;
1-ethyl-6-(3-nitrophenyl)-4-oxo-1,4-dihydronicotinic acid;
1-ethyl-6-(3-aminophenyl)-4-oxo-1,4-dihydronicotinic acid;
6-[4-(dimethylamino)-phenyl]-1-methoxy-4-oxo-1,4-dihydronicotinic acid;
1-butoxy-6-[4-(dimethylamino)-phenyl]-4-oxo-1,4-dihydronicotinic acid;
6-(4-acetamidophenyl)-1-ethyl-4-oxo-1,4-dihydronicotinic acid;
1-ethyl-4-oxo-6-[3-(trifluoromethyl)-phenyl]-1,4-dihydronicotinic acid;
1-ethyl-6-[4-(4-methyl-1-piperazinyl)-phenyl]-4-oxo-1,4-dihydronicotinic acid;
1-ethyl-6-(p-methoxystyryl)-4-oxo-1,4-dihydronicotinic acid;
1-ethyl-4-oxo-6-(4-pyridyl)-1,4-dihydronicotinic acid.

7. A process for the manufacture of the 4-pyridone-3-carboxylic acid derivatives claimed in claim 1, which process comprises
(a) dehydrogenating a 1,4,5,6-tetrahydronicotinic acid derivative of the general formula

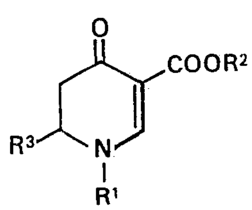

II

wherein $R^1$, $R^2$ and $R^3$ have the significances given in claim 1,
in the 5,6-position,
or
(b) modifying a substituent $R^3$ in a manner known per se within the definitions given above, and, if desired, converting the compound of formula I obtained into a salt.

8. A process for the manufacture of the 4-pyridone-3-carboxylic acid derivatives claimed in claim 2, which process comprises

(a) dehydrogenating a 1,4,5,6-tetrahydronicotinic acid derivative of the general formula

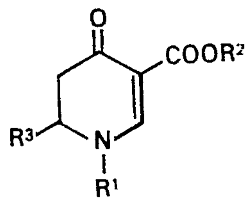

II

wherein $R^1$, $R^2$ and $R^3$ have the significances given in claim 2,
in the 5,6-position,
or
(b) modifying a substituent $R^3$ in a manner known per se within the definitions given above, and, if desired, converting the compound of formula I obtained into a salt.

9. Pharmaceuticals on the basis of a 4-pyridone-3-carboxylic acid derivative as claimed in any one of claims 1-6.

10. Antibacterial agents on the basis of a 4-pyridone-3-carboxylic acid derivative as claimed in any one of claims 1-6.

11. Central nervous system-stimulating agents on the basis of a 4-pyridone-3-carboxylic acid derivative as claimed in any one of claims 1-6.

12. 4-Pyridone-3-carboxylic acid derivatives as set forth in any one of claims 1-6 for use as pharmaceutical agents.

13. 4-Pyridone-3-carboxylic acid derivatives as set forth in any one of claims 1-6 for use as antibacterial agents.

14. 4-Pyridone-3-carboxylic acid derivatives as set forth in any one of claims 1-6 for use as central nervous system-stimulating agents.